# EUROPEAN PATENT SPECIFICATION

(11) **EP 1 414 299 B1**
(45) Date of publication and mention of the grant of the patent: **04.05.2011**
(21) Application number: 02736574.1
(22) Date of filing: 15.05.2002
(51) Int. Cl.: A61K 47/48, A61P 35/00

(54) **METHOD FOR MAKING A HOMOGENOUS DOXORUBICIN -TRANSFERRIN CONJUGATE**
VERFAHREN ZUR HERSTELLUNG EINES HOMOGENEN DOXORUBICIN-TRANSFERRIN KOJUGATES
METHODE DE PREPARATION D'UN CONJUGUE HOMOGENE DOXORUBICINE-TRANSFERRINE

(30) Priority: 15.05.2001 US 290684 P; 17.10.2001 US 329535 P
(43) Date of publication of application: 06.05.2004
(73) Proprietor: Faulk Pharmaceuticals, Inc., Indianapolis, IN 46240 (US)
(72) Inventor: FAULK, Ward Page, Indianapolis, IN 46240 (US)
(74) Representative: Murray, Adrian D'Coligny
(86) International application number: PCT/US2002/011891
(87) International publication number: WO 2002/091991

(56) References cited:
- WO-A-02/094271
- WO-A-03/032899
- WO-A1-95/03831
- US-A- 4 886 780
- US-A- 5 393 737
- US-A- 6 020 316
- DATABASE CA [Online] CHEMICAL ABSTRACTS SERVICE, COLUMBUS, OHIO, US; OHKAWA, KIYOSHI: "Trials of molecular targeting to overcome multidrug resistance" XP002309083 retrieved from STN Database accession no. 2000:151715 & TOKYO JIKEIKAI IKA DAIGAKU ZASSHI , 115(1), 1-9 CODEN: TJIDAH; ISSN: 0375-9172, 2000,
- WANG F. ET AL.: 'Doxorubicin-gallium-transferin conjugate overcomes multidrug resistance: evidence for drug accumulation in the nucleus of drug resistant MCF-7/ADR cells' ANTICANCER RESEARCH vol. 20, no. 2A, 2000, pages 799 - 808, XP002966761
- FAULK W.P. ET AL.: 'Preliminary clinical study of transferrin-adriamycin conjugate for drug delivery to acute Leukemia patients' MOLECULAR BIOTHERAPY vol. 2, 1990, pages 57 - 60, XP002966762
- SACHDEVA M.S.: 'Drug targeting systems for cancer chemotherapy' EXPERT OPINION INVESTIGATION DRUGS vol. 7, no. 11, 1998, pages 1849 - 1864, XP000990640
- FAULK W.P. ET AL: 'Transferrin Conjugates of Adriamycin Are Cytotoxic without Intercalating Nuclear DNA' THE JOURNAL OF BIOLOGICAL CHEMISTRY vol. 267, 08 May 1992, pages 9437 - 9442
- CHANG-JING G.Y. ET AL: 'Killing of Human Tumor Cells in Culture with Adryamycin Conjugates of human Transferrin' CLINICAL IMMUNOLOGY AND IMMUNOPATHOLOGY vol. 32, 01 January 1984, pages 1 - 11
- BERCZI A. ET AL: 'Adriamycin Conjugates of Human Transferrin Bind Transferrin Receptors and Kill K562 and HL60 Cells' ARCHIVES OF BIOCHEMISTRY AND BIOPHYSICS vol. 300, no. 1, 01 January 1993, pages 356 - 363, XP24753472
- BARABAS ET AL: 'Transferrin Conjugates of Adryamicin are Cytotoxic without intercalating Nuclear DNA' J. BIOL.CHEM vol. 267, 08 May 1992, pages 9437 - 9442

## Description

### FIELD OF THE INVENTION

This invention relates generally to the field of bio-affecting materials and more specifically to homogeneous protein-drug conjugates, the method of their making and the method of their use.

### BACKGROUND OF THE INVENTION

Two of the most devastating problems in cancer treatment are drug-toxicity, which debilitates patients, and drug-resistance, which is normally countered with even higher drug dosages and thus amplifies the problem of drug-toxicity, often resulting in death. One way to solve the problem of drug-toxicity is to deliver drugs so they are targeted only to cancer cells. Many researchers are working to develop antibodies to deliver drugs, and this approach holds promise, but antibodies are not without problems.

For example, antibodies often bind to normal tissues, and they also can damage blood vessels (e.g., vascular leak syndrome) and cause dangerous allergic reactions (e.g. anaphylaxis).

Research is also progressing in connection with the use of conjugates of transferrin and anticancer drugs as described in US patent numbers 5,108,987; 5,000,935; 4,895,714; and 4,886,780 as well as by Wang et al., Anticancer Research 20:799-808 (2000) and Faulk et al., Molecular Biotherapy 1990 Mar 2(1), 57-60.

The inventions described in these patents do not use antibodies. Instead, they use a protein found in normal human blood. This protein is transferrin, which delivers iron. Normal cells rarely require iron, but cancer cells require large amounts of iron to maintain their pathologically increased rates of metabolism.

Because cancer cells require more iron, they have transferrin receptors substantially permanently on their surfaces, whereas normal cells do not. These inventions exploits these receptors by administering anticancer drugs bonded to transferrin, which delivers the drugs substantially only to transferrin receptors on the surface of cancer cells.

Drug targeting spares normal cells, requires less drug, and significantly diminishes drug-toxicity. In contrast, when anticancer drugs are administered without being targeted, they kill normal cells as well as cancer cells. They are particularly toxic to cells of the immune system and to the system responsible for blood clotting. Thus, infections and bleeding are principal complications of chemotherapy in cancer patients. These complications require expensive services, hospitalizations, intensive care, and life-support systems, which are uncomfortable and expensive for the patient. These problems are largely preventable by using targeted delivery systems.

The problem of drug-toxicity consumes huge blocks of the time of doctors and nurses, and is responsible for much of the cost of cancer care. For example, it is commonly understood that about 70% of calls to oncologists relate to a problem of drug-toxicity. Today there is no satisfactory way to treat drug-toxicity, except to use less drug. Targeted delivery allows the use of less drug, because more of the administered drug is delivered specifically to cancer cells rather than being nonspecifically distributed around the body. In this sense, targeted delivery is like shooting with a rifle, while conventional delivery is like shooting with a shotgun. A solution to the problem of drug-toxicity will dramatically transform chemotherapy in cancer patients. It is a purpose of this invention to reduce such adverse effects of chemotherapy.

The problem of drug-resistance is equally as serious as the problem of drug-toxicity. This problem is typified by a patient diagnosed with cancer who is treated and responds with a symptomless remission that lasts many months, and who later sees the cancer returns in a form that no longer responds to any known drug. This scenario of drug-resistance is all too common. Yet today there is no satisfactory solution, except the use of larger amounts of more powerful drugs that in turn can cause serious drug-toxicity problems, often resulting in death. A solution to the problem of drug-resistance would significantly diminish the problem of drug-toxicity. Transferrin-targeted drug delivery can overcome the problem of drug-resistance. Thus, another purpose of the present invention is to resolve the issue of painful and expensive deaths from drug-resistant cancers.

The effectiveness of proteins conjugated with bio-affecting molecules has been demonstrated and is described in the US patents mentioned above. It has been determined; however, that the efficiency of such conjugates in treating stressed cells, such as cancer cells, is reduced by the presence of agglutinated conjugates or by the presence of conjugates of a bio-affecting molecule with protein fragments or with two or three protein molecules and is greatly enhanced when the protein to bio-affecting molecule ratio is closer to 1:1. Obtaining conjugates of higher efficiency has, in the past, been a slow, tedious and expensive process that requires separating a fraction of conjugate having the desired average ratio of bio-affecting molecule to protein from a larger sample comprising such molecules conjugated with protein fragments, with a plurality of proteins and proteins conjugated with a plurality of bio-affecting molecules. Using homogeneous protein-drug conjugates in which the protein component carries a predetermined number of bio-affecting molecules can more effectively kill both drug-resistant and drug-sensitive cancer cells. The past expense and inefficiency inherent in producing useful conjugates in a useful volume has been a problem for the commercialization of such conjugates and for their widespread use in medicine. There is a need for a substantially homogeneous drug-protein conjugate and for a method of making such a conjugate that is more efficient, more precise and less costly. It is one purpose of this invention to provide such a homogeneous conjugate made by a more efficient method.

### DESCRIPTION OF THE RELATED ART

The first report of transferrin receptors on human cancer cells was by Faulk and colleagues in 1980 (1). This was followed by many reports of transferrin receptors in different types of human cancers (2), as seen in the following Table.

| Tumor Studied | References | Tumor Studied | Reference |
|---|---|---|---|
| Breast | 1, 3 | Gastrointestinal | 10 |
| Leukemia | 4, 5 | Ovary | 11 |
| Lung | 6 | Non-Hodgkin's lymphoma | 12 |
| Brain | 7 | Lymphoma/melanoma | 13,14 |
| Liver | 8 | Nasopharyngeal | 15 |
| Bladder | 9 | Cervix | 16 |

### Transferrin Receptors on Normal and on Cancer Cells.

No single study has asked if all human cancers have up-regulated transferrin receptors, or if all normal cells have down regulated transferrin receptors, but data from many quarters suggest that the answer to both questions is yes. For example, immature erythrocytes (i.e., normoblasts and reticulocytes) have transferrin receptors on their surfaces, but mature erythrocytes do not (17). Circulating monocytes also do not have up-regulated transferrin receptors (18), and macrophages, including Kupffer cells, acquire most of their iron by a transferrin-independent method of erythrophagocytosis (19). In fact, in vivo studies indicate that virtually no iron enters the reticuloendothelial system from plasma transferrin (for review, see reference 20). Macrophage transferrin receptors are down regulated by cytokines such as gamma interferon (21), presumably as a mechanism of iron-restriction to kill intracellular parasites (22).

In resting lymphocytes, not only are transferrin receptors down regulated, but the gene for the transferrin receptor is not measurable (23). In contrast, stimulated lymphocytes up-regulate transferrin receptors in late G₁, (24). Receptor expression occurs subsequent to expression of the c-myc proto-oncogene and following up-regulation of IL-2 receptor (25), and is accompanied by a measurable increase in iron-regulatory protein binding activity (26), which stabilizes transferrin receptor mRNA (27). This is true for both T and B lymphocytes (28), and is an IL-2-dependent response (29).

Cell stimulation resulting in the up regulation of receptors for transferrin is known to result from stress experienced, for example, by cells invaded by a viral factor and by cancer cells.

Up-and-down regulation of transferrin receptors for normal and tumor cells has been shown by studies of antigen or lectin stimulation (i.e., receptor up-regulation), and by studies of differentiation models (30-33) using retinoic acid (i.e., receptor down-regulation). Base-line data from these experimental models suggest that these receptors are down regulated from the plasma membranes of most normal, adult, resting human cells (34). Exceptions are the circulatory barrier systems, which include the materno-fetal barrier with its transferrin receptor-rich syncytiotrophoblast (35); the blood-brain barrier with its transferrin receptor-rich capillary endothelial cells (36); and, the blood-testis barrier with its transferrin receptor-rich Sertoli cells (37).

### Mechanism of Cell Killing by Transferrin-Drug Conjugates.

Transferrin-doxorubicin conjugates bind to plasma membranes by sequentially employing two reactions; initially the transferrin component is bound by transferrin receptors, after which the doxorubicin component is bound by the lipid bilayer, primarily by interacting with cardiolipin and charged phosphates (5 8). Thus, bound through protein and phospholipid receptors, the conjugates are positioned to activate signal transduction pathways by receptor dimerization, lateral mobility and cytoplasmic calcium mobilization (61).

One mechanism involved in the killing of tumor cells by transferrin-doxorubicin conjugates is the inhibition of plasma membrane redox enzymes, particularly the inhibition of NADH-oxidase (62). Inhibition of NADH-oxidase causes cell death (63), and doxorubicin is an efficient inhibitor of this enzyme (64,65). Transferrin-doxorubicin conjugates inhibit NADH-oxidase (66), as well as down-stream reactions initiated by NADH oxidation, such as loss of electrons and exchange of protons through the sodium-hydrogen antiport (67).

A second mechanism of cell killing by transferrin-doxorubicin conjugates involves the molecular control of transferrin receptors. For example, chelation of microenviromental iron initiates apoptosis in tumor cells but not in normal resting cells (68), and such chelation enhances significantly the cytotoxic effect of cytosine arabinoside (69). Drug-resistant cells are much more sensitive to iron restriction, due to their inability to stabilize transferrin receptor mRNA, and excess iron destabilizes transferrin receptor mRNA more effectively in drug-resistant than in drug-sensitive cells (70).

A third mechanism involves redox-active products of oxidative stress (71). For example, nitric oxide disassembles the iron-sulfur cluster, allowing iron-regulatory proteins to bind and protect iron-response elements (72). Hydrogen peroxide causes the same effect (i.e., up-regulation of transferrin receptors), but transferrin receptors are down regulated by the nitrosium ion, which causes nitrosylation of thiol groups within the iron-sulfur cluster (73). In summary, there are at least three mechanisms involved in the killing of cells by transferrin-doxorubicin conjugates.

Until now, the widespread treatment by such mechanisms of cells under stress and having up regulated transferrin receptors has effectively been blocked by the expense and time required to isolate a fraction of a protein-drug conjugate that contains a controlled ratio of protein to drug with substantially no dimers, polymers or fractions of protein from a reaction product that is likely to contain mostly such undesirable fractions. (As mentioned elsewhere, proteins such as transferrin that are agglutinated, fractionated, or the like will not interact correctly with transferrin receptors, if at all.) The inefficiency of the past process has made the treatment of cancer cells and of cells infected with a virus by these mechanisms economically unattractive.

### Transferrin-Drug Conjugates in Laboratory Animals

The efficacy of transferrin-drug conjugates has been investigated in several animal models. For example, conjugates of transferrin with diphtheria toxin decrease xenografted gliomas in nude mice by 95% on day 14, and the gliomas did not recur by day 30 (74). Also, glutaraldehyde-prepared transferrin-doxorubicin conjugates have been found to rescue nude mice from death by human mesothelioma cells, significantly prolonging life compared to animals treated only with doxorubicin (75). In addition, transferrin has been coupled to herpes simplex thymidine kinase by using biotinstreptavidin technology , and these conjugates significantly prolonged life in nude mice inoculated with metastasizing K562 tumor cells (76). Finally, the maximum tolerated dose of human transferrin-doxorubicin conjugates in nude mice has been found to be 20 mg/kg (iv) for conjugates and only 8 mg/kg (iv) for free drug (41).

### Transferrin-Drug Conjugates in Human Patients

There are two clinical reports of transferrin-drug conjugates. The first, published in 1990, was a preliminary study of seven acute leukemia patients treated intravenously with 1 mg/day of glutaraldehyde-prepared transferrin-doxorubicin conjugates for 5 days. With these low doses, there were no toxic effects and the number of leukemic cells in peripheral blood of the 7 patients decreased by 86% within 10-days following therapy (77). In addition, there was no extension of disease as assessed by examination of bone marrow biopsies before and after treatment.

The second, published by the NIH in 1997, involved 15 patients with recurrent brain cancers treated with thioether-bonded transferrin conjugates of a genetic mutant of diphtheria toxin (44). The conjugates were delivered by high-flow interstitial microinfusion, which has been shown to produce effective perfusion of radiolabeled transferrin in primate brains with minimal inflammatory responses (78). Magnetic resonance imaging revealed at least a 50% reduction in tumor volume in 9 of the 15 patients, including 2 cases of complete remission (44).

There is an unpublished clinical study of 23 patients with advanced ovarian cancer who were randomized into test (12 patients) and placebo (11 patients) groups. The test group received transferrin-doxorubicin conjugates equivalent to 1 mg doxorubicin per day on days 15 through 19 of monthly treatment cycles. A significant difference was revealed by Cox regression estimates of survival rates for patients treated with transferrin-doxorubicin conjugates when the time between diagnosis and randomization was 18 months.

Another unpublished study is a 22-year old male with metastatic disease from a sarcoma of his right atrium who was treated by conventional protocols without response. His lungs were filled with metastatic lesions when his physician father obtained an IND from the FDA for the use of transferrin-doxorubicin conjugates, and treatment was begun in August, 2000. By November, the lungs were substantially cleared of metastatic lesions, and by January there was no radiological evidence of tumor. He presently (August 2001) is active, receiving only transferrin-doxorubicin.

The targeted delivery of drugs has the advantage of increasing efficacy while using less drug, thereby decreasing toxicity and causing less damage to normal cells, all of which effectively decrease costs and increase the quality of patient care. Targeted delivery also avoids drug-resistance, which is activated by the non-specific entrance of drugs into cells (79). Because transferrin-drug conjugates enter cells specifically by employing a receptor-specific pathway (80,81), they are trafficked around drug-resistance mechanisms, such as efflux pumps in resistant cells.

It was reported in 1992 that transferrin-doxorubicin conjugates effectively kill multi-drug resistant cells (82). This finding was confirmed in 1993 (83), and was extended to several types of drug-resistant cells in 1994 (84), 1996 (85) and 2000 (86).

### Preparation of Transferrin-Drug Conjugates.

A method for the preparation of transferrin-doxorubicin conjugates was published first in 1984 (38), following which there have been many reports of methods for the preparation of transferrin-drug conjugates, some of which are listed in the following Table.

| Transferrin Label | Method Used | References | Transferrin Label | Method Used | References |
|---|---|---|---|---|---|
| Doxorubicin | Glutaraldehyde | 38,39,40 | Titanium | Carbonate | 48 |
| Doxorubicin | Maleimide | 41 | Insulin | Disulfide | 49 |
| Mitomycin C | Glutaryl Spacer | 42 | Gallium | Carbonate | 50 |
| Neocarzinostatin | Succinimide | 43 | Platinum | Methionine | 51 |
| Diphtheria Toxin | Thioester | 44 | Saporin/ricin | Succinimide | 52 |
| Chlorambucil | Maleimide | 45 | Ruthenium | Bicarbonate | 53 |
| Paclitaxol | Glutaraldehyde | 46 | Growth Factor | Fusion Protein | 54 |
| Daunorubicin | Glutualdehyde | 47 | HIV Protease | Recombinant | 55 |

Transferrin conjugates of doxorubicin can be prepared by using glutaraldehyde-mediated Schiff base formation (56,57), which forms an acid-resistant bond between epsilon-amino lysine groups of transferrin and the 3'amino position of doxorubicin. Such conjugates of doxorubicin can kill cancer cells through a plasma membrane-mediated mechanisms (for review, see reference 58). Although DNA intercalation is an established mechanism of cell death by doxorubicin, immobilized doxorubicin on carriers, such as dextran, activate plasma membrane-mediated mechanisms to kill cells (59,60). It thus appears that conjugates of doxorubicin with transferrin kill cells by activating plasma membrane-mediated mechanisms that involve both doxorubicin and transferrin receptors.

The ability of non-antibody proteins such as transferrin conjugated with anticancer drugs to target cancer cells and to kill drug-resistant cells efficiently has been found to depend on the molecular ratio of drug-to-transferrin. Excessive loading of a protein such as transferrin with bio-affecting molecules is believed to interfere with the protein's ability to dock with receptors. Under-loading of such a protein is believed to result in receptors being filled with proteins that do not carry drugs, a phenomenon known as blocking. Contemporary techniques for the preparation of transferrin-drug conjugates do not allow for the production of conjugates with predetermined ratios. Presently available procedures provide a heterogeneity of conjugates, including a large percentage that are either excessively loaded or that are under loaded. Isolation of a relatively small useful fraction from the presently available manufacturing procedures results in very low yields of clinically usable molecules and very high production costs. The expense involved causes the production and use of an otherwise effective cure for cancers and other conditions causing cells to undergo stress to be economically unattractive, thus denying the benefits of the material to most patients. There is a need for a high volume and lower cost method of making such conjugates.

### SUMMARY OF THE INVENTION

It is apparent that drugs combined with targeting agents have a generic possibility of changing how drugs are delivered, as well as a specific possibility of changing how drugs are delivered to cancer patients. However, it is a problem that it hitherto has not been possible to synthesize large amounts of homogeneous conjugates with predetermined and consistent numbers of drug molecules per molecule of protein. This and other problems with known methods for making and using conjugates of transferrin with doxorubicin are solved by the present invention.

According to a first aspect of the present invention, there is provided a method for making a transferrin-doxorubicin conjugate having a predetermined ratio of doxorubicin to transferrin comprising the steps of adding a saline solution of doxorubicin, dropwise, into a saline solution of glutaraldehyde linker material containing DMSO to a final concentration of a 1:1 molar ratio of doxorubicin to glutaraldehyde in a manner that effectively results in the addition of one doxorubicin molecule to one glutaraldehyde molecule; and adding the doxorubicin/glutaraldehyde combination to transferrin in a manner that results in a conjugate having a predetermined ratio of transferrin to doxorubicin; wherein said transferrin-doxorubicin conjugate is free of dimers, trimers and aggregates.

According to a second aspect of the present invention, there is provided a homogeneous material comprising a transferrin conjugated with doxorubicin in a predetermined ratio of doxorubicin to transferrin molecules obtainable from the above first aspect of the present invention, wherein said transferrin is attracted to receptors on target cells.

According to a third aspect of the present invention, there is provided a method for making a conjugate having a predetermined doxorubicin:transferrin ratio, comprising adding a solution of a glutaraldehyde linker molecule drop-wise to solution of DMSO to produce a first intermediate product, adding a solution of doxorubicin dropwise to said first intermediate product to a final concentration of a 1:1 molar ratio of doxorubicin to glutaraldehyde to link one doxorubicin molecule to one glutaraldehyde linker molecule in a doxorubicin/glutaraldehyde combination to produce a second intermediate product; and adding the second intermediate product to a protein targeting agent to produce a third intermediate product having the predetermined drug: protein ratio, adding a linker scavenging agent to said third intermediate product to produce a fourth intermediate product, and filtering said fourth intermediate product to obtain a conjugate having a predetermined doxorubicin:transferrin ratio, wherein said conjugate is free of dimers, trimers and aggregates.

### DETAILED DESCRIPTION OF THE INVENTION

The presently preferred method comprises adding an anticancer drug, to a linker such that there is a controlled ratio of drug connected to each linker molecule. The drug-linker material is added to transferrin in amounts to achieve a desired molar ratio. In the present process the linker is glutaraldehyde. Glutaraldehyde was selected as a linker because its presents only two reaction sites and because its reaction kinetics favor the attachment of only one bio-affecting molecule to each linker molecule. Excess glutaraldehyde may be scavenged with, for example, ethanolamine following formation of the conjugate.

The present invention relates to homogeneous conjugates with a predetermined and consistent number of antitumor agent or other bio-affecting molecule per molecule of protein targeting agent. The targeting agent is transferrin. The anti-tumor agent is doxorubicin.

The invention also relates to efficient and economical methods for preparing substantially homogeneous conjugates having a predetermined and consistent number of antitumor agents or other bio-affecting molecules per molecule of protein targeting agent. This process substantially reduces, and in most cases virtually eliminates the production of polymers and dimers of transferrin or aggregates of transferrin drug conjugates, thus yielding a narrow range of drug-protein ratios. The present invention substantially decreases production costs and increases efficiency while increasing the effectiveness of the conjugate in medical applications. The substantially homogeneous conjugates according to the present invention result from a process beginning with the formation of reactive drug-linker complexes. In this illustration of the process the bio-affecting material is doxorubicin and the linker is glutaraldehyde.

The synthesis of large amounts of homogeneous transferrin-doxorubicin conjugates with predetermined molecular ratios was done stoichiometrically by employing the only amino group of doxorubicin (DOX, which is at the 3' amino position, to react with one of the two reactive groups on glutaraldehyde (GLU). Thus, the first step was drop-wise addition of a saline solution of DOX into a saline solution of GLU containing a solvent such as DMSO or another suitable cryopreservative, to a final concentration of a 1:1 molar ratio of DOX-to-GLU. The resulting solution of DOX-GLU was stirred three hours at room temperature in the dark.

The molarities of DOX and GLU were the same in the above reaction in order to produce a final solution of DOX-GLU that contains neither free DOX nor free GLU. However, there is the possibility of free GLU in solution if one GLU reacts with two DOX to produce DOX-GLU-DOX, but this possibility is minimized by the mass action kinetics generated by drop-wise addition of monovalent DOX into the solution of bivalent GLU. The volumes of these reactants are not restricted, so large amounts of homogeneous DOX-GLU can be prepared.

The second step in the conjugation reaction was drop-wise addition of DOX-GLU into a saline solution of transferrin (TRF). The TRF can be either iron-free (apotransferrin) or iron-saturated (holo-transferrin). The desired molar ratio of DOX to TRF was obtained by appropriately adjusting the volume of TRF. The resulting solution of TRF-GLU-DOX was stirred for 20 hours at room temperature in the dark. Unlike the reaction of DOX with GLU, the reaction of DOX-GLU with TRF is not restricted to one binding site, for the GLU component of DOX-GLU can react with any one of several epsilon-amino lysine groups in the TRF molecule.

The number of DOX molecules bound to TRF was determined in the second step. For example, if the starting ratio of DOX-GLU to TRF was 7.2:1.0, the final solution of TRF-GLU-DOX would have contained 2.5 molecules of DOX per molecule of TRF. However, if the starting ratio of DOX-GLU to TRF was 4.0:1.0, the final solution of TRF-GLU-DOX would have contained 1.4 molecules of DOX per molecule of TRF. Similarly, if the starting ratio of DOX-GLU to TRF was 2.5:1.0, the final solution of TRF-GLU-DOX would have contained 0.9 molecules of DOX per molecule of TRF. In this way, large amounts of TRF-GLU-DOX with predetermined ratios of DOX-to-TRF can be provided according to the need.

Further steps in the conjugation reaction were the addition of ethanolamine or another substance suitable for scavenging any excess linker, followed by centrifugation and dialysis. Although reactions with DOX and TRF theoretically consume all of the GLU, ethanolamine was added to the final reaction mixture to bind any available GLU. This reaction was allowed to continue for 30 minutes in the dark. The final solution was centrifuged at 2000 rpm for 10 minutes, dialyzed twice for 6 hours in a 100-fold excess of saline and three times in the same excess of Hepes buffered saline, and the resulting TRF-GLU-DOX conjugates were ready for use.

### Biochemical Characterization of the Conjugates:

By using HPLC and polyacrylamide gel electrophoresis as described in (39), the homogeneity of TRF-GLU-DOX conjugates can be determined. Also, by using spectrophotometry as described in (89), the molecular ratio of DOX-to-TRF can be determined. These techniques repeatedly have revealed a consistent homogeneity of the TRF-GLU-DOX conjugates. In addition, chromatography is not required in the preparation of these conjugates, because there are no aggregates or fragments. This allows for the preparation of large volumes of homogeneous transferrin-drug conjugates, which increases yields and decreases costs.

The expenses caused by losses of TRF and DOX in other types of transferrin-drug conjugates have been an impediment to their use. For example, yields of DOX and TRF are decreased by using procedures such as thiolation (44) that alter the drug and/or protein. Yields also are decreased by using solvent systems (86) and by chromatography used to prepare acid-stable and acid-labile linkages (41). The GLU bond between DOX and TRF is acid-stable (89), and yields of DOX and TRF in TRF-DOX conjugates prepared according to this invention are high. Indeed, compared to other procedures (38, 39, 40), the yield for TRF is nearly doubled (90% vs 50%), and the yield for DOX is increased 5-fold.

None of the previously known approaches to the preparation of transferrin-doxorubicin conjugates are capable of producing large amounts of homogeneous conjugates with predetermined ratios of the number of drug molecules per molecule of transferrin. In addition, the other approaches employ chromatography to eliminate aggregates and to harvest fractions that are enriched in homogeneous conjugates. These procedures decrease yields, increase costs, and lack the ability to predetermine molecular ratios.

### Characterizing the Conjugates

After the pure drug-protein conjugates are isolated, they are characterized by polyacrylamide gel electrophoresis to determine their molecular weight, and the number of drug molecules per protein molecule is determined. The exact number of drug molecules per transferrin molecule can be determined, using any suitable technique including spectrophotometric techniques. A functional drug:protein ratio is between about 0.1:1.0 to 3.0:1.0 (Berczi et al., Arch Biochem Biophys 1993; 300:356). The conjugates are checked to determine if they bind to receptors on the surface of tumor cells, and to determine if the conjugates kill cancer cells but not normal cells. Only conjugates that bind to cancer cells and not to normal cells are selected for toxicity tests using drug-sensitive and drug-resistant cancer cells. The binding studies can be done by using flow cytometry or any other suitable method, and the killing studies can be done by using microculture techniques to determine the concentration of free drug required to kill 50% of a culture of cancer cells compared to the concentration of drug in the drug-protein conjugates required to kill the same number of cancer cells. When testing the heat sensitizer conjugates, the toxicity test is done by using the MIT tetrazolium colorimetric assay (Visitica et al., Cancer Res 1994; 51: 2515). These toxicity tests determine the most potent transferrin sensitizer ratio and the optimum concentration of conjugate for maximum heat sensitization of drug sensitive and drug resistant cells. Approximately 10-fold more free drug compared to drug in the drug-protein conjugate is required to kill the same number of cells.

After the drug-protein conjugate has been prepared, purified, characterized and validated for cellular binding and killing properties, and, when the binding and killing experiments show that the conjugate binds to and kills cancer but not normal cells, the conjugate is then aliquoted and sterilized. The sterilization process can be done by any suitable method including exposure to irradiation, such as by using a cesium irradiator, or by using Millipore filtration techniques.

As used in the present document, the term "homogeneous conjugates" means that the conjugates can be used without further purification to remove protein dimers, polymers or aggregates. In other words, little or no protein dimers, polymers or aggregates are present.

The substantially homogeneous conjugates according to the present invention are administered to an animal in an effective amount. In treating cancer, an effective amount includes an amount effective to: reduce the size of a tumor; slow the growth of a tumor; prevent or inhibit metastases; or increase the life expectancy of the affected animal. The present invention provides for a method of treating a variety of cancers including leukemia, breast cancer, ovarian cancer, pancreatic cancer, lung cancer, bladder cancer, gastrointestinal cancer, nasopharyngeal cancer, cervical cancer, myeloma, lymphoma/melanoma, glioma, or astrocytoma. The dosage for the homogeneous conjugates can be determined taking into account the age, weight and condition of the patient and the pharmacokinetics of the anti-tumor agent. The amount of the homogeneous conjugate required for effective treatment will be less than the amount required using the anti-tumor agent alone. For example, the dosage of a conjugate of transferrin-doxorubicin is expected to be between 0.5-50mg per 28 day period for a 150 pound (68 kg) person. The dosage can be divided and administered as smaller doses at varying intervals during the 28 day period.

The pharmaceutical compositions of the invention can be administered by a number of routes, including orally, topically, rectally, ocularly, vaginally, by the pulmonary route, for instance, by use of an aerosol, or parenterally, including intramuscularly, subcutaneously, intraperitoneally, intra-arterially or intravenously. The compositions can be administered alone, or can be combined with a pharmaceutically-acceptable carrier or excipient according to standard pharmaceutical practice. For the oral mode of administration, the compositions can be used in the form of tablets, capsules, lozenges, troches, powders, syrups, elixirs, aqueous solutions and suspensions. For parenteral administration, sterile solutions of the homogeneous conjugate are usually prepared, and the pHs of the solutions are suitably adjusted and buffered. For intravenous use, the total concentration of solutes should be controlled to render the preparation isotonic. For ocular administration, ointments or droppable liquids may be delivered by ocular delivery systems known to the art such as applicators or eye droppers. For pulmonary administration, diluents and/or carriers will be selected to be appropriate to allow the formation of an aerosol. It is preferred that the conjugate of the present invention be administered parenterally, i.e. intravenously or intraperitoneally, by infusion or injection.

Preferred embodiments of the present invention are described below.

### Example 1

### Preparation of a homogeneous transferrin-doxorubicin conjugate

The synthesis of large amounts of homogeneous transferrin-doxorubicin conjugates with predetermined molecular ratios was done stoichiometrically by employing the only amino group of doxorubicin (DOX), which is at the 3' amino position, to react with one of the two reactive groups on glutaraldehyde (GLU). Thus, the first step was drop-wise addition of a saline solution of DOX into a saline solution of GLU containing a solvent such as DMSO to a final concentration of a 1:1 1 molar ratio of DOX-to-GLU. The resulting solution of DOX-GLU was stirred three hours at room temperature in the dark.

The molarities of DOX and GLU were the same in the above reaction in order to produce a final solution of DOX-GLU that contains neither free DOX nor free GLU. However, there is the possibility of free GLU in solution if one GLU reacts with two DOX to produce DOX-GLU-DOX, but this possibility is minimized by the mass action kinetics generated by drop-wise addition of monovalent DOX into the solution of bivalent GLU. The volumes of these reactants are not restricted, so large amounts of homogeneous DOX-GLU can be prepared.

The second step in the conjugation reaction was drop-wise addition of DOX-GLU into a saline solution of transferrin (TRF). The TRF can be either iron-free (apotransferrin) or iron-saturated (holo-transferrin). The desired molar ratio of DOX to TRF was obtained by appropriately adjusting the volume of TRF. The resulting solution of TRF-GLU-DOX was stirred for 20 hours at room temperature in the dark. Unlike the reaction of DOX with GLU, the reaction of DOX-GLU with TRF is not restricted to one binding site, for the GLU component of DOX-GLU can react with any one of several epsilon-amino lysine groups in the TRF molecule.

The number of DOX molecules bound to TRF was determined in the second step. For example, if the starting ratio of DOX-GLU to TRF was 7.2:1.0, the final solution of TRF-GLU-DOX would have contained 2.5 molecules of DOX per molecule of TRF. However, if the starting ratio of DOX-GLU to TRF was 4.0:1.0, the final solution of TRF-GLU-DOX would have contained 1.4 molecules of DOX per molecule of TRF. Similarly, if the starting ratio of DOX-GLU to TRF was 2.5:1.0, the final solution of TRF-GLU-DOX would have contained 0.9 molecules of DOX per molecule of TRF. In this way, large amounts of TRF-GLU-DOX with predetermined ratios of DOX-to-TRF can be provided according to the need.

Further steps in the conjugation reaction were the addition of ethanolamine, followed by centrifugation and dialysis. Although reactions with DOX and TRF theoretically consume all of the GLU, ethanolamine was added to the final reaction mixture to bind any available GLU. This reaction was allowed to continue for 30 minutes in the dark. The final solution was centrifuged at 2000 rpm for 10 minutes, dialyzed twice for 6 hours in a 100-fold excess of saline and three times in the same excess of Hepes buffered saline, and the resulting TRF-GLU-DOX conjugates were ready for use. Biochemical Characterization of the Conjugates:
By using HPLC and polyacrylamide gel electrophoresis as described in (39), the homogeneity of TRF-GLU-DOX conjugates can be determined. Also, by using spectrophotometry as described in (89), the molecular ratio of DOX-to-TRF can be determined. These techniques repeatedly have revealed a consistent homogeneity of the TRF-GLU-DOX conjugates. In addition, chromatography is not required in the preparation of these conjugates, because there are no aggregates or fragments. This allows for the preparation of large volumes of homogeneous transferrin-drug conjugates, which increases yields and decreases costs.

The expenses caused by losses of TRF and DOX in other types of transferrin-drug conjugates have been an impediment to their use. For example, yields of DOX and TRF are decreased by using procedures such as thiolation (44) that alter the drug and/or protein. Yields also are decreased by using solvent systems (86) and by chromatography used to prepare acid-stable and acid-labile linkages (41). The GLU bond between DOX and TRF is acid-stable (89), and yields of DOX and TRF in TRF-DOX conjugates prepared according to this invention are high. Indeed, compared to other procedures (38, 39, 40), the yield for TRF is nearly doubled (90% vs 50%), and the yield for DOX is increased 5-fold.

None of the previously known approaches to the preparation of transferrin-doxorubicin conjugates are capable of producing large amounts of substantially homogeneous conjugates with predetermined ratios of the number of drug molecules per molecule of transferrin. In addition, the other approaches employ chromatography to eliminate aggregates and to harvest fractions that are enriched in homogeneous conjugates. These procedures decrease yields, increase costs, and lack the ability to predetermine molecular ratios.

### In Vitro Characterization of the Conjugates:

Conjugates of TRF-GLU-DOX prepared according to this invention have the ability to bind and kill cancer cells but not normal cells. By using flow cytometry as described in (39), these conjugates have been shown to bind cultured human cancer cells and not normal peripheral blood lymphocytes. Also, by using cell culture techniques as described in (39), the TRF-DOX conjugates have been shown to kill cultured human cancer cells but not normal cells. These procedures also serve as quality controls for the homogenous TRF-GLU-DOX conjugates described herein.

The TRF-GLU-DOX conjugates described in this patent also have the ability to kill drug-resistant cancer cells. Earlier experimental data indicated that other conjugates of TRF with anticancer drugs can kill multi-drug resistant human cancer cells by binding transferrin receptors (82), and that such resistant cells have been shown to have more transferrin receptors on their surfaces than do drug-sensitive cells (90). Though presently unpublished and unreported, the TRF-GLU-DOX conjugates described herein have been found to uniformly bind and kill drug-resistant cells. Thus, homogeneous TRF-GLU-DOX conjugates with predetermined molecular ratios, such as those described herein, provide clinically useful molecules for killing both drug-resistant and drug-sensitive cancer cells by uniformly and consistently binding transferrin receptors.

### In Vivo Characterization of the Conjugates:

Nude mice xenografted with human drug-resistant human mesothelioma cancer cells survived significantly longer when they were treated with TRF-DOX conjugates than when they were treated with free DOX (75), providing proof-of-principle that transferrin-drug conjugates kill drug-resistant human cancer cells in a mouse model. However, these results are dependent on the ability to produce large amounts of homogeneous TRF-DOX conjugates containing a predetermined number of DOX molecules per molecule of TRF.

In unpublished experiments using TRF-DOX conjugates prepared as described herein, nude mice xenografted with lethal doses of drug-sensitive and drug-resistant human cancer cells survived significantly longer when treated with TRF-DOX conjugates than when treated with placebo (i.e., albumin), unconjugated TRF or free DOX. In these experiments, mice with drug-resistant tumors received the same dose of TRF-DOX as mice with drug-sensitive tumors.

### REFERENCES

### (References in bold type are from Dr. Faulk's laboratory)

1. Faulk WP, Hsi BL and Stevens PJ. Transferrin and transferrin receptors in carcinoma of the breast. Lancet 1980; ii: 390-392
2. Faulk WP, Harats H and Berczi A. Transferrin receptor growth control in normal and abnormal cells. In: Oxidoreduction at the Plasma Membrane. Vol 1. (eds., FL Crane, JD Morre and H Low) CRC Press, Boca Raton, FL, 1990; pp. 205-224.
3. Yang DC, Wang F, Elliott RL and Head JF. Expression of transferrin receptor and ferritin H-chain mRNA are associated with clinical and histopathological prognostic indicators in breast cancer. Anticancer Res 2001; 21: 541-549.
4. Yeh CJG, Taylor CG and Faulk WP. Targeting of cytotoxic drugs by transferrin receptors: Selective killing of acute myelogenous leukemia cells. Protides Biol Fluids 1984; 32: 441-444.
5. Barnett D, Wilson GA, Lawrence AC and Buckley GA. Transferrin receptor expression in the leukaemias and lymphoproliferative disorders. Clin Lab Haematol 1987; 9: 361-70.
6. Whitney JF, Clark JM, Griffin TW, Gautam S and Leslie KO. Transferrin receptor expression in nonsmall cell lung cancer. Histopathologic and clinical correlates. Cancer 1995; 76: 20-25.
7. Recht L, Torres CO, Smith TW, Raso V and Griffin TW. Transferrin receptor in normal and neoplastic brain tissue: implications for brain-tumor immunotherapy. J Neurosurg 1990; 72: 941-945.
8. Sciot R, Paterson AC, van Eyken P, Callea F, Kew MC and Desmet VJ. Transferrin receptor expression in human hepatocellular carcinoma: an immunohistochemical study of 34 cases. Histopathol 1988; 12: 53-63
9. Seymour GJ, Walsh MD, Lavin MF, Strutton G and Gardiner RA. Transferrin receptor expression by human bladder transitional cell carcinomas. Urol Res 1987; 15: 341-344.
10. Lindholn ML, Lindberg LA, Vilja P, Puolakka VM, Nordling S, Schroder T and Schroder J. Expression of the human transferrin receptor in subrenal capsule assay in the mouse. J Surg Oncol 1988; 38: 57-62.
11. Hereiz HA and Bayoumi FA. Evaluation of diagnosis of ovarian malignancy using immunohistochemical technique. J Egyptian Public Hlth Assoc 1992; 67: 697-707.
12. Medeiros LJ, Picker LJ, Homing SJ and Warnke RA. Transferrin receptor expression by non-Hodgkin's lymphomas. Correlation with morphologic grade and survival. Cancer 1988; 61: 1844-185.
13. Yeh CJG, Taylor C and Faulk WP. Transferrin binding by peripheral blood mononuclear cells in human lymphomas, myelomas and leukemias. Vox Sanguinis 1984; 46: 217-223.
14. Soyer HP, Smolle J, Tome R and Kerl H. Transferrin receptor expression in normal skin and in various cutaneous tumors. J Cutaneous Pathol 1987; 14: 1-5.
15. Pannccio M, Zalcberg JR, Thompson CH, Leyden JM, SullivanJR, Lichtenstein Mand McKenzie IF. Heterogeneity of the human transferrin receptor and use of anti-transferrin receptor antibodies to detect tumors in vivo. Immunol & Cell Biol 1987; 65: 461-472.
16. Farley J, Loup D, Nelson M, Miller MJ, Taylor R and Gray K. Transferrin in normal and neoplastic endocervical tissues: distribution and receptor expression. Analyst & Quant Cytol & Histol 1998; 20: 238-249.
17. Lesley J, Hyman R, Schulte R and Trotter J. Expression of transferrin receptor on murine hematopoietic progenitors. Cell Immunol 1984; 83: 14-25.
18. Testa U, Pelosi E and Peschle C. The transferrin receptor. Crit Rev Oncogen 1993; 4: 241-276.
19. Bothwell TA, Charlton RW, Cook JD and Finch CA. Iron Metabolism in Man, Blackwell Scientific, Oxford, 1979.
20. Ponka P and Lok CN. The transferrin receptor: role in health and disease. Int J Biochem Cell Biol 1999; 31: 1111-1137.
21. Hamilton TA, Gray PW and Adams DO. Expression of the transferrin receptor on murine peritoneal macrophages is modulated by in vitro treatment with interferon gamma. Cell Immunol 1984; 89: 478-488.
22. Byrd TF and Horowitz MA. Interferon gamma-activated human monocytes downregulate transferrin receptors and inhibits the intracellular multiplication of Legionella. pneumophila by limiting the availability of iron. J Clin Invest 1989; 83: 1457-1465.
23. Kronke M, Leonard W, Depper JM and Greene WC. Sequential expression of genes involved in human T lymphocyte growth and differentiation. J Exp Med 1985; 161: 1593-1598.
24. Galbraith RM and Galbraith GM. Expression of transferrin receptors on mitogen-stimulated human peripheral blood lymphocytes: relation to cellular activation and related metabolic events. Immunology 1983; 133: 703-710.
25. Neckers LM and Cossman J. Transferrin receptor induction in mitogen-stimulated human T lymphocytes is required for DNA synthesis and cell division and is regulated by interleukin 2. Proc Nat Acad Sci USA 1983; 80: 3494-3498.
26. Testa U, Kuhn L, Petrini M, Quaranta MT, Pelosi E and Peschle C. Differential regulation of iron regulatory element-binding protein(s) in cell extracts of activated lymphocytes versus monocytes-macrophages. J Biol Chem 1991; 266: 3925-3930.
27. Seiser C, Texieira S and Kuhn LC. Interleukin-2-dependent transcriptional and post-transcriptional regulation of transferrin receptor mRNA. J Biol Chem 1993; 268: 13,074-13,080.
28. Neckers LM, Yenokida G and James SP. The role of the transferrin receptor in human B lymphocyte activation. J Immunol 1984; 133: 2437-2441.
29. Neckers LM and Trepel JB. Transferrin receptor expression and the control of cell growth. Cancer Invest 1986; 4: 461-470.
30. Yeh CJG, Papamichail M and Faulk WP. Loss of transferrin receptors following induced differentiation of HL-60 promyelocytic leukemia cells. Exper Cell Res 1982; 138: 429-431.
31. Barker KA and Newburger PE. Relationships between the cell cycle and the expression c-myc and transferrin receptor genes during induced myeloid differentiation. Exper Cell Res 1990; 186: 1-5.
32. Klausner RD, Rouault TA and Harford JB. Regulating the fate of mRNA: the control of cellular iron metabolism Cell 1993; 72: 19-28.
33. Haile DJ. Regulation of genes of iron metabolism by the iron-response proteins. Am J Med Sciences 1999; 318: 230-240.
34. Gatter KC, Brown G, Trowbridge IS, Woolston RE and Mason DY. Transferrin receptors in human tissues: their distribution and possible clinical relevance. J Clin Pathol 1983; 36: 539-545.
35. Faulk WP and Hunt JS. Human placentae: view from an immunological bias. Am J Reprod Immunol 1990; 21: 108-113.
36. Broadwell RD, Baker-Caims BJ, Friden PM, Oliver C and Villegas JC. Transcytosis of protein through the mammalian cerebral epithelium and endothelium III. Receptor mediated transcytosis through the blood-brain barrier of blood-borne transferrin and antibody against transferrin receptor. Exp Neurol 1996; 142: 47-65.
37. Sylvester SR and Griswold MD. The testicular iron shuttle: A "nurse" function of the Sartoli cells. J Androl 1994; 15: 381-385.
38. Yeh CJG and Faulk WP. Killing of human tumor cells in culture with doxorubicin conjugates of human transferrin. Clin Immunol Immunopath 1984; 32: 1-11.
39. Berczi A, Barabas K, Sizensky JA and Faulk WP. Doxorubicin conjugates of human transferrin bind transferrin receptors and kill K562 and HL60 cells. Arch Biochem Biophys 1993; 300: 356-363.
40. Lai BT, Gao JP and Lanka KW. Mechanism of action and spectrum of cell lines sensitive to a doxorubicin-transferrin conjugate. Cancer Chemother & Pharmacol 1998; 41: 155-160.
41. Kratz F, Beyer U, Roth T, Tarasova N, Collery P, Lechenault F, Cazabat A, Schumacher P, Unger C and Falken U. Transferrin conjugates of doxorubicin: synthesis, characterization, cellular uptake, and in vitro efficacy. J Pharm Sciences 1998; 87: 338-346.
42. Tanaka T, Kaneo Y and Miyashita M. Synthesis of transferrin-mitomycin C conjugate as a receptor-mediated drug targeting system. Biol Pharm Bull 1996; 19: 774-777.
43. Sasaki K, Kohgo Y, Kato J, Kondo H and Niitsu Y. Intracellular metabolism and cytotoxicity of transferrin-neocarzinostatin conjugates of differing molar ratios. Jpn J Cancer Res 1993; 84: 191-196.
44. Laske DW, Youle RJ and Oldfield EH. Tumor regression with regional distribution of the targeted toxin TF-CRM107 in patients with malignant brain tumors. Nature Med 1997; 3: 1362-1368.
45. Beyer U, Roth T, Schumacher P, Maier G, Unold A, Frahm AW, Fiebig HH, Unger C and Kratz F. Synthesis and in vitro efficacy of transferrin conjugates of the anticancer drug chlorambucil. J Med Chem 1998; 41: 2701-2708.
46. Bicamumpaka E and Page M. In vitro cytotoxicity of paclitaxel-transferrin conjugate on H69 cells. Oncol Reports 1998; 5: 1381-1383.
47. Lemieux P, Page M and Noel C. In vivo cytotoxicity and antineoplastic activity of a transferrin-daunorubicin conjugate. In Vivo 1992; 6: 621-627.
48. Guo M, Sun H, McArdle HJ, Gambling L and Sadler PJ. Ti(IV) uptake and release by human serum transferrin and recognition of Ti(IV)-transferrin by cancer cells: understanding the mechanism of action of the anticancer drug titanocene dichloride. Biochem 2000; 39: 10023-10033.
49. Shah D and Shen WC. Transcellular delivery of an insulin-transferrin conjugate in enterocyte-like Caco-2 cells. J Pharm Sciences 1996; 85: 1306-1311.
50. Drobyski WR, Ul-Haq R, Majewski D and Chitambar CR. Modulation of in vitro and in vivo T-cell responses by transferrin-gallium and gallium nitrate. Blood 1996; 88: 3056-3064.
51. Hoshino T, Misaki M, Yamamoto M, Shimizu H, Ogawa Y and Toguchi H. In vitro cytotoxicities and in vivo distribution of transferrin-platinum(II) complex. J Pharm Sciences 1995; 84: 216-221.
52. Ippoliti R, Ginobbi P, Lendaro E, D'Agostino I, Ombres D, Benedetti PA, Brunori M and Citro G. The effect of monensin and chloroquine on the endocytosis and toxicity of chioneric toxins. Cell Mol Life Sci 1998; 54: 866-875.
53. Kratz F, Hartmann F, Keppler B and Messor L. The binding properties of two antitumor ruthenium(III) complexes to apotransferrin. J Biol Chem 1994; 269: 2581-2588.
54. Park E, Starzyk RM, McGrath JP, Lee T, George J, Schutz AJ, Lynch P and Putney SD. Production and characterization of fusion proteins containing transferrin and nerve growth factor. J Drug Targeting 1998; 6: 53-64.
55. Ali SA, Joao HC, Hammerschmid F, Eder J and Steinkasserer A. Transferrin Trojan Horses as a rational approach for biological delivery of therapeutic peptide domains. J Biol Chem 1999; 274: 24066-24073.
56. Peters K and Richards FM. Chemical cross-linking: reagents and problems in studies of membrane structure. Annu Rev Biochem 1977; 46: 523-551.
57. Rhodes J. Evidence for an intercellular covalent reaction essential in antigen-specific T cell activation. J Immunol 1989; 143: 1482-1489.
58. Tritton TR. Cell surface actions of doxorubicin. Pharmacol & Therapeutics 1991: 49: 293-309.
59. Maestre N, Tritton TR, Laurent G and Jaffrezou JP. Cell surface-directed interaction of anthracyclines leads to cytotoxicity and nuclear factor kappaB activation but not apoptosis signaling. Cancer Res 2001; 61: 2558-2561.
60. Fong WF, Lam W, Yang M and Wong JT-F. Partial synergism between dextran-conjugated doxorubicin and cancer drugs on the killing of multidrug resistant KB-V1 cells. Anticancer Res 1996; 16: 3773-3778.
61. Sainte-Marie J, Lafont V, Pecheur EI, Favero J, Philippot JR and Bienvenue A. Transferrin receptor functions as a signal-transduction molecule for its own recycling via increases in the internal Ca++ concentration. Euro J Biochem 1997; 250: 689-697.
62. Crane FL, Low H, Sun IL, Morre DJ and Faulk WP. Interaction between oxidoreductase, transferrin receptor and channels in the plasma membrane. In: Growth Factors from Genes to Clinical Applications (eds, VR Sara, K Hall and H Low) Raven Press, New York, 1990; pp. 228-239.
63. Sun IL, Navas P, Crane FL, Morre DJ and Low H. Diferric transferrin reductase in the plasma membrane is inhibited by doxorubicin. Biochem Int 1987; 14: 119-127.
64. Faulk WP, Harats H, McIntyre JA, Berczi A, Sun IL and Crane FL. Recent advances in cancer research: Drug targeting without the use of monoclonal antibodies. Am J Reprod Immunol 1989; 21: 151-154.
65. Morre DJ, Kim C, Paulik M, Morre DM and Faulk WP. Is the drug-response NADH-oxidase of the cancer cell plasma membrane a molecular target for doxorubicin? Bioenerg Biomembr 1997; 29: 269-280.
66. Sun IL, Sun EE, Crane FL, Morre DJ and Faulk WP Inhibition of transplasma membrane electron transport by transferrin-doxorubicin conjugates. Biochim membrane electron transport by transferrin-doxorubicin conjugates. Biochim Biophy Acta 1992; 1105: 84-88.
67. Faulk WP, Barabas K, Sun IL and Crane FL. Transferrin-doxorubicin conjugates which inhibit tumor cell proliferation without interaction with DNA inhibit plasma membrane oxidoreductase and proton release in K562 cells. Biochem Int 1991; 25: 815-822.
68. Hileti D, Panayiotidis P and Hoffbrand V. Iron chelators induce apoptosis in proliferating cells. Brit J Haematol 1995; 89: 181-187.
69. Leardi A, Caraglia M, Selleri C, Pepe S, Pizzi C, Notaro R, Fabbrocini A, De Lorenzo S, Musico M, Abbruzzese A, Bianco A and Tagliaferri P. Desferioxamine increases iron depletion and apoptosis induced by ara-C of human myeloid leukemic cells. Brit J Haematol 1998; 102: 746-752.
70. Barabas K, Miller SJ and Faulk WP. Regulation of transferrin receptor mRNA stability in drug-sensitive and drug-resistant cancer cells. To be submitted for publication, 2001.
71. Hentze MW and Kuhn LC. Molecular control of vertebrate iron-metabolism: mRNA-based regulatory circuits operated by iron, nitric oxide and oxidative stress. Proc Natl Acad Sci USA 1996; 93: 8175-8182.
72. Pantapoulos K and Hentze MW. Rapid responses to oxidative stress mediated by iron regulatory protein. EMBO J 1995; 14: 2917-1924.
73. Richardson DR, Naumannova V, Nagy E and Ponka P. The effect of redox-related species of nitrogen monoxide on transferrin and iron uptake and cellular proliferation of erythroleukemia (K562) cells. Blood 1995; 86: 3211-3219.
74. Laske DW, Ilercil O, Akbasak A, Youle RJ and Oldfield EH. Efficacy of direct intratumoral therapy with targeted protein toxins for solid human gliomas in nude mice. J Neurosurg 1994; 80: 520-526.
75. Singh M, Atwal H and Micetich R. Transferrin directed delivery of doxorubicin to human cells. Anticancer Res 1998; 18(3A): 1423-1427.
76. Sato Y, Yamauchi N, Takahashi M, Sasaki K, Fukaura J, Neda H, Fujii S, Hirayma M, Itoh Y, Koshita Y, Kogawa K, Kato J, Sakamaki S and Niitsu Y. In vivo gene delivery to tumor cells by transferrin-streptavidin-DNA conjugate. FASEB Journal 2000; 14: 2108-2118.
77. Faulk WP, Taylor CG, Yeh G and McIntyre JA. Preliminary clinical study of transferrin-doxorubicin conjugate for drug delivery to acute leukemia patients. Mol Biother 1990; 2: 57-60.
78. Laske DW, Morrison PF, Lieberman DM, Carthesy ME, Reynolds JC, Stewart-Henney PA, Koong SS, Cummins A, Paik CH and Oldfield EH. Chronic interstitial infusion of protein to primate brain: determination of drug distribution and clearance with single-photon emission computerized tomography imaging. J Neurosurg 1997; 87: 586-594.
79. Marbeuf-Gueye C, Ettori D, Priebe W, Kozlowski H and Garnier-Suillerot A. Correlation between the kinetics of anthracycline uptake and the resistance factor in cancer cells expressing the multidrug resistance protein or the P-glycoprotein. Biochem Biophy Acta 1999; 1450: 374-384.
80. Klausner RD, vanReuswoude J, Ashwell G, Kempf C, Schechter AN, Dean A and Bridges K. Receptor-mediated endocytosis of transferrin in K562 cells. J Biol Chem 1983; 258: 4715-4724.
81. Berczi A, Ruthner M, Szuts V, Fritzer M, Schweinzer E and Goldenberg H. Influence of conjugation of doxorubicin to transferrin on the iron uptake by K562 cells via receptor-mediated endocytosis. Euro J Biochem 1993; 213: 427-436.
82. Fritzer M, Barabas K, Szuts V, Berczi A, Szekeres T, Faulk WP and Goldenberg H. Cytotoxicity of a transferrin-doxorubicin conjugate to anthracylcine resistant cells. Int J Cancer 1992; 52: 619-623.
83. Hatano T, Ohkawa K and Matsuda M. Cytotoxic effect of the protein-doxorubicin conjugates on the multidrug-resistant human myelogenous leukemia cell line, K562, in vitro. Tumor Biology 1993; 14: 288-294.
84. Lemieux P and Page M. Sensitivity of multidrug-resistant MCF-7 cells to a transferrin-doxorubicin conjugate. Anticancer Res 1994; 14(2A): 397-403.
85. Fritzer M, Szekeres T, Szuts V, Jraayam HN and Goldenberg H. Cytotoxic effects of a doxorubicin-transferrin conjugate in multidrug-resistant KB cells. Biochem Pharm 1996; 51: 489-493.
86. Wang F, Jiang X, Yang DC, Elliot RL and Head JF. Doxorubicin-gallium-transferrin conjugate overcomes multidrug resistance: evidence for drug accumulation in the nucleus of drug resistant MCF-7/ADR cells. Anticancer Res 2000; 20: 799-808.
87. Soma CE, Dubemet C and Barratt G. Ability of doxorubicin-loaded nanoparticles to overcome multidrug resistance of tumor cells after their capture by macrophages. Pharm Res 1999; 16: 1710-1716.
88. Mazel M, Clair P, Rousselle C, Vidal P, Scherrmann J-M, Mathieu D and Temsamani J. Doxorubicin-peptide conjugates overcome multidrug resistance. Anti-Cancer Drugs 2001; 12: 107-116.
89. Barabas K, Sizensky JA and Faulk WP. Transferrin conjugates of doxorubicin are cytotoxic without intercalating nuclear DNA. J Biol Chem 1992; 267: 9437-9442.
90. Barabas K and Faulk WP. Transferrin receptors associate with drug resistance in cancer cells. Biochem Biophys Res Com 1993; 197: 702-708.

## Claims

1. A method for making a transferrin-doxorubicin conjugate having a predetermined ratio of doxorubicin to transferrin comprising the steps of:
a) adding a saline solution of doxorubicin, dropwise, into a saline solution of glutaraldehyde linker material containing DMSO to a final concentration of a 1:1 molar ratio of doxorubicin to glutaraldehyde in a manner that effectively results in the addition of one doxorubicin molecule to one glutaraldehyde molecule; and
b) adding the doxorubicin/glutaraldehyde combination to transferrin in a manner that results in a conjugate having a predetermined ratio of transferrin to doxorubicin;
wherein said transferrin-doxorubicin conjugate is free of dimers, trimers and aggregates.

2. A method according to Claim I, further comprising scavenging any excess glutaraldehyde linker.

3. A homogeneous material comprising a transferrin conjugated with doxorubicin in a predetermined ratio of doxorubicin to transferrin molecules obtainable from the method of Claim 1 or 2, wherein said transferrin is attracted to receptors on target cells.

4. A material according to Claim 3, wherein the predetermined ratio of doxorubicin molecules to transferrin molecules is between 0.1:1.0 and 8:1.0.

5. A material according to Claim 4, wherein the predetermined ratio of doxorubicin molecules to transferrin molecules is between 0.1:1.0 and 4:1.0.

6. A material according to Claim 4, wherein the predetermined ratio of doxorubicin molecules to transferrin molecules is between 0.2:1.0 and 8:1.0.

7. A material according to any one of Claims 3 to 6 wherein said material is free of dimers, trimers and aggregates.

8. A reagent kit for the treatment of tumors, comprising iron-bearing transferrin, and the material of any one of Claims 3 to 7.

9. A method for making a conjugate having a predetermined doxorubicinaransferrin ratio, comprising
a) adding a solution of a glutaraldehyde linker molecule drop-wise to solution of DMSO to produce a first intermediate product,
b) adding a solution of doxorubicin dropwise to said first intermediate product to a final concentration of a 1:1 molar ratio of doxorubicin to glutaraldehyde to link one doxorubicin molecule to one glutaraldehyde linker molecule in a doxorubicin/glutaraldehyde combination to produce a second intermediate product; and
c) adding the second intermediate product to a protein targeting agent to produce a third intermediate product having the predetermined drug:
protein ratio,
d) adding a linker scavenging agent to said third intermediate product to produce a fourth intermediate product, and
e) filtering said fourth intermediate product to obtain a conjugate having a predetermined doxorubicin:transferrin ratio,
wherein said conjugate is free of dimers, trimers and aggregates.

## Patentansprüche

1. Verfahren zur Herstellung eines Transferrin-Doxorubicin-Konjugats, das ein vorherbestimmtes Verhältnis von Doxorubicin zu Transferrin hat, das die folgenden Schritte umfasst:
a) tropfenweise Zugabe einer Kochsalzlösung von Doxorubicin in eine Kochsalzlösung von Glutaraldehyd-Linkersubstanz, die DMSO enthält, bis zu einer Endkonzentration von einem molaren Verhältnis von 1 : 1 von Doxorubicin zu Glutaraldehyd auf eine Weise, die effektiv zur Addition von einem Doxorubicin-Molekül an ein Glutaraldehyd-Molekül führt; und
b) Zugabe der Doxorubicin/Glutaraldehyd-Kombination zu Transferrin auf eine Weise, die zu einem Konjugat führt, das ein vorherbestimmtes Verhältnis von Transferrin zu Doxorubicin hat;
worin genanntes Transferrin-Doxorubicin-Konjugat frei von Dimeren, Trimeren und Aggregaten ist.

2. Verfahren nach Anspruch 1, das weiterhin das Abfangen von jeglichem Überschuss an Glutaraldehyd-Linker umfasst.

3. Homogener Stoff, der ein mit Doxorubicin konjugiertes Transferrin in einem vorherbestimmten Verhältnis von Doxorubicin- zu Transferrin-Molekülen, der aus dem Verfahren nach Anspruch 1 oder 2 erhalten werden kann, umfasst, worin genanntes Transferrin von Rezeptoren auf Zielzellen angezogen wird.

4. Stoff nach Anspruch 3, worin das vorherbestimmte Verhältnis von Doxorubicin-Molekülen zu Transferrin-Molekülen zwischen 0,1 : 1,0 und 8 : 1,0 beträgt.

5. Stoff nach Anspruch 4, worin das vorherbestimmte Verhältnis von Doxorubicin-Molekülen zu Transferrin-Molekülen zwischen 0,1 : 1,0 und 4 : 1,0 beträgt.

6. Stoff nach Anspruch 4, worin das vorherbestimmte Verhältnis von Doxorubicin-Molekülen zu Transferrin-Molekülen zwischen 0,2 : 1,0 und 8 : 1,0 beträgt.

7. Stoff nach einem der Ansprüche 3 bis 6, worin genannter Stoff frei von Dimeren, Trimeren und Aggregaten ist.

8. Reagenz-Kit zur Behandlung von Tumoren, der Eisen-enthaltendes Transferrin und den Stoff nach einem der Ansprüche 3 bis 7 umfasst.

9. Verfahren zur Herstellung eines Konjugats, das ein vorherbestimmtes Doxorubicin : Transferrin-Verhältnis hat, das Folgendes umfasst:
a) tropfenweise Zugabe einer Lösung eines Glutaraldehyd-Linkermoleküls zu einer Lösung von DMSO, um ein erstes Zwischenprodukt herzustellen,
b) tropfenweise Zugabe einer Lösung von Doxorubicin zum genannten ersten Zwischenprodukt bis zu einer Endkonzentration von einem molaren Verhältnis von 1 : 1 von Doxorubicin zu Glutaraldehyd, um ein Doxorubicin-Molekül mit einem Glutaraldehyd-Linkermolekül zu einer Doxorubicin/Glutaraldehyd-Kombination zu verknüpfen, um ein zweites Zwischenprodukt herzustellen; und
c) Zugabe des zweiten Zwischenprodukts zu einem auf Protein zielgerichteten Mittel, um ein drittes Zwischenprodukt herzustellen, das das vorherbestimmte Wirkstoff : Protein-Verhältnis hat,
d) Zugabe eines Linker-abfangenden Mittels zum genannten dritten Zwischenprodukt, um ein viertes Zwischenprodukt herzustellen, und
e) Filtrieren von genanntem vierten Zwischenprodukt, um ein Konjugat zu erhalten, das ein vorherbestimmtes Doxorubicin : Transferrin-Verhältnis hat,
worin genanntes Konjugat frei von Dimeren, Trimeren und Aggregaten ist.

## Revendications

1. Méthode de fabrication d'un conjugué de transferrine-doxorubicine présentant un rapport prédéterminé doxorubicine à transferrine, comprenant les étapes consistant à :
a) ajouter goutte à goutte une solution saline de doxorubicine dans une solution saline de substance liante de glutaraldéhyde contenant du DMSO jusqu'à une concentration finale ayant un rapport molaire 1:1 doxorubicin à glutaraldéhyde, d'une manière qui conduit efficacement à l'addition d'une molécule de doxorubicine à une molécule de glutaraldéhyde ; et
b) ajouter la combinaison doxorubicine/glutaraldéhyde à la transferrine d'une manière qui conduit à un conjugué présentant un rapport prédéterminé transferrine à doxorubicine ;
ledit conjugué transferrine/doxorubicine étant exempt de dimères, de trimères et d'agrégats.

2. Méthode selon la revendication 1, comprenant en outre la désactivation de tout excès de liant glutaraldéhyde.

3. Substance homogène comprenant une transferrine conjuguée à une doxorubicine selon un rapport prédéterminé des molécules doxorubicine à transferrine pouvant être obtenu à partir de la méthode selon la revendication 1 ou 2, ladite transferrine étant attirée vers des récepteurs sur des cellules cibles.

4. Substance selon la revendication 3, dans laquelle le rapport prédéterminé des molécules de doxorubicine aux molécules de transferrine est compris entre 0,1:1,0 et 8:1,0.

5. Substance selon la revendication 4, dans laquelle le rapport prédéterminé des molécules de doxorubicine aux molécules de transferrine est compris entre 0,1:1,0 et 4:1,0.

6. Substance selon la revendication 4, dans laquelle le rapport prédéterminé des molécules de doxorubicine aux molécules de transferrine est compris entre 0,2:1,0 et 8:1,0.

7. Substance selon l'une quelconque des revendications 3 à 6, ladite substance étant exempte de dimères, de trimères et d'agrégats.

8. Kit réactif pour le traitement de tumeurs, comprenant une transferrine portant du fer et la substance selon l'une quelconque des revendications 3 à 7.

9. Méthode de fabrication d'un conjugué ayant un rapport prédéterminé doxorubicine à transferrine, comprenant les étapes consistant à :
a) ajouter goutte à goutte une solution d'une molécule liante de glutaraldéhyde à une solution de DMSO afin de produire un premier produit intermédiaire ;
b) ajouter goutte à goutte une solution de doxorubicine audit premier produit intermédiaire jusqu'à une concentration finale ayant un rapport molaire 1:1 doxorubicine à glutaraldéhyde afin de lier une molécule de doxorubicine à une molécule liante de glutaraldéhyde dans une combinaison doxorubicine/glutaraldéhyde afin de produire un deuxième produit intermédiaire ; et
c) ajouter le deuxième produit intermédiaire à un agent ciblant les protéines afin de produire un troisième produit intermédiaire présentant le rapport prédéterminé médicament:protéine ;
d) ajouter un agent de désactivation du liant audit troisième produit intermédiaire afin de produire un quatrième produit intermédiaire ; et
e) filtrer ledit quatrième produit intermédiaire afin d'obtenir un conjugué présentant un rapport prédéterminé doxorubicine à transferrine ;
ledit conjugué étant exempt de dimères, de trimères et d'agrégats.
